(19) Europäisches Patentamt
European Patent Office
Office européen des brevets

(11) Publication number: 0 100 660
B1

(12) **EUROPEAN PATENT SPECIFICATION**

(45) Date of publication of patent specification: **06.04.88**

(51) Int. Cl.$^4$: **C 12 N 11/00**

(21) Application number: **83304391.2**

(22) Date of filing: **29.07.83**

(54) **A bioreactor and a process for the production thereof.**

(30) Priority: **29.07.82 JP 133055/82**
**29.12.82 JP 230159/82**
**31.03.83 JP 57508/83**

(43) Date of publication of application:
**15.02.84 Bulletin 84/07**

(45) Publication of the grant of the patent:
**06.04.88 Bulletin 88/14**

(84) Designated Contracting States:
**DE FR GB IT**

(56) References cited:
**DE-A-2 042 976**
**FR-A-2 350 874**
**FR-A-2 407 937**

**Smith-D'Ans, Anorganische Chemie (1967), p. 441**
**Iler, The Chemistry of Silica (1979), p. 516**

(73) Proprietor: **SHIMADZU CORPORATION**
**378, Ichinofunairi-cho Kawaramachi Dori Nijo Sagaru**
**Nakagho-ku Kyoto 604 (JP)**

(72) Inventor: **Shotaro, Oka**
**20-1 Nakajyo Mozume-cho**
**Muko-shi Kyoto-fu, 617 (JP)**
Inventor: **Shu, Tahara**
**303 Izumiden Heights 1-18-7 Tenjin**
**Nagaokakyo-shi Kyoto-fu, 617 (JP)**
Inventor: **Hiroyoshi, Minakuchi**
**141 Kanze-cho Imadegawa-agaru Omiya-dori**
**Kamigyo-ku Kyoto-shi, 602 (JP)**
Inventor: **Hajime, Karatani**
**401 Ojuen 17 Entomi-cho Shogoin**
**Sakyo-ku Kyoto-shi, 606 (JP)**

(74) Representative: **Shipley, Warwick Grenville Michael**
**VENNER, SHIPLEY & CO. 368 City Road**
**London EC1V 2QA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 100 660 B1

Courier Press, Leamington Spa, England.

**Description**

The present invention relates to a bioreactor and a process for production thereof and, more particularly to a bioreactor suited for use of analysis, diagnosis and synthesis which comprises immobilizing a peptide-containing compound such as a biocatalyst, etc. to a specific carrier, as well as a process for production thereof.

Recently immobilized glasses which comprise immobilizing peptide-containing compounds such as enzyme or the like to a glass carrier have been employed as bioreactors for use of analysis, diagnosis or synthesis. As a process for producing these bioreactors, a process which comprises treating the surface of a $SiO_2$ type glass previously obtained by a fusion technique with an alkali to form hydroxy groups therein, introducing, e.g., aminoalkyl groups, into the hydroxy group and then immobilizing an enzyme or the like, is known and has been reduced to practical use. To introduce functional groups such as the aforesaid aminoalkyl groups, a method using various silane coupling agents is known.

In the prior art process, however, a step of forming hydroxy groups at the glass surface was required. In addition, the amount of the hydroxy group per unit area which could be formed at this step was limited and an amount of the peptide-containing compound to be immobilized was also limited. It was thus difficult to obtain bioreactors having an increased amount of the peptide-containing compound and having a high activity.

DE—A—2042976 discloses a method of stabilising antigens and antibodies on an inorganic carrier and a composite prepared by this method. However, there is no disclosure of a hydrolysate of a metal alkoxide as the inorganic carrier.

FR—A—2350874 discloses a hydrophilic complex gel prepared from a tetraalkoxysilane and a watersoluble polymer selected from polyvinyl alcohol, gelatine and carboxymethylcellulose. It is concerned with a kind of gel-entrapping method which has the disadvantage that entrapped enzyme molecules cannot exert their activity. The enzyme is added to the components while they are still in a sol state, followed by hydrolysis. This makes it difficult to maintain the pH value, resulting in a reduction of activity or even an inactivation of the enzyme material.

It is an object of the present invention to overcome the foregoing problems.

On the other hand, a process for preparing metal oxide glasses which comprises hydrolysing metal alkoxides as raw materials and treating the resulting glass-like gels at high temperatures is known in the field of glass production and, in particular, in the field of optical glass production.

We have now found that by the use of glass-like gels formed by using metal alkoxides and/or alkoxysilanes as raw materials and hydrolysing the metal alkoxides and/or alkoxysilanes under mild conditions, i.e. intermediates for the production of glass from the aforesaid metal alkoxides and/or alkoxysilanes, as carriers for immobilizing peptide-containing compounds, bioreactors can easily be obtained, without requiring any step of introducing hydroxy groups, in which the immobilized amount of peptide-containing compounds per unit carrier is increased.

Thus, according to the present invention, there is provided a bioreactor comprising a peptide-containing compound chemically bound to a carrier, characterised in that the carrier is a glass-like gel comprising a metal hydroxy compound and/or a condensation product thereof obtainable by hydrolysis of a metal alkoxide and/or of an alkoxysilane.

Furthermore, the present invention provides a bioreactor comprising a peptide-containing compound chemically bound to a carrier, characterised in that the carrier is a glass-like gel comprising a metal hydroxy compound and/or a condensation product thereof, the hydroxy groups of which are partially substituted by fluorine atoms, said carrier being obtainable by hydrolysis of a metal alkoxide and/or of an alkoxysilane and substitution of some of the resulting hydroxy groups by fluorine atoms.

The present invention will now be described in more detail, with reference to the accompanying drawings, in which:

Figure 1 is a graph illustrating the activity of an immobilized enzyme of the present invention with the passage of time;

Figure 2 is a graph illustrating the activity of an immobilized enzyme of the present invention, together with that of a comparative example;

Figure 3 is a graph illustrating the relationship between the relative activities and the mole ratios of HF/$Si(OC_2H_5)4$ of immobilized enzymes of the present invention;

Figure 4 is a graph illustrating the analytical chart with ESCA of a glass-like gel obtained according to Example 4;

Figure 5 is a graph illustrating the analytical chart with TG of glass-like gels obtained according to Example 5;

Figure 6 is a graph illustrating the analytical chart with DTG of glass-like gels obtained according to Example 5;

Figure 7 is an enlarged perspective view of a glass-like gel obtained according to Example 6; and

Figure 8 is a graph illustrating the relative activity of the immobilized enzymes according to Example 7.

The metal alkoxides used in the present invention include the various metal alkoxides known in the field of glass production or ceramic production. It is to be understood that, within the scope of the present invention, the term metal alkoxides includes silicon alkoxides. Specific examples of the metal alkoxides

include lower alkoxysilanes, lower alkoxytitaniums, lower alkoxyaluminiums and lower alkoxyvanadiums, such as $Si(OCH_3)_4$ $Si(OC_2H_5)_4$, $Ti(OC_3H_7)_4$, $V(OC_2H_5)_3$ and $Al(OC_3H_7)_3$. Of these, it is generally preferred to use lower alkoxysilanes. A mixture of two or more of such compounds can also be employed. Sodium methoxide and like monovalent metal alkoxides can be used in admixture with the above-mentioned tri- and tetravelent metal alkoxides.

The bioreactors of the present invention are characterized in that glass-like gels obtained by hydrolysis of the aforesaid metal alkoxides are employed as carriers for immobilization. Furthermore, the term "bioreactor" used herein means a peptide-containing compound immobilized on a material, which may be suitably formed on a supporting substrate.

In the case of using, e.g., lower alkoxy silanes, it is generally appropriate that the hydrolysis of the lower alkoxy silanes be carried out in an aqueous solvent in an acidic system under mild conditions. In this case, it is preferred that the hydrolysis be proceeded while evaporating the solvent off and avoiding the elevation of the temperature as less as possible such that glass-like gels be formed in the state where high degree of condensation of the metal hydroxide compound be prevented. In this case, it is appropriate to use a volatile hydrophilic solvent as the aqueous solvent. For example, water-containing lower alcohols such as water-containing methanol, water-containing ethanol, etc. are advantageously employed. In some occasion, the water may not be contained since the water may be supplied from the atmosphere. It is also possible to effect the hydrolysis in water alone; in this case, however, there is a danger that the hydrolysis might proceed vigorously and render the system heterogeneous and such is disadvantageous for drying the gels.

It is appropriate that the pH of the solvent be generally adjusted to an acidic area (pH of about 1 to about 3), generally by adding small quantities of inorganic acids such as hydrochloric acid, etc. The glass-like gel used in the present invention is usually isolated by allowing the system to stand in the air, after adding a metal alkoxide in such an aqueous solvent and thoroughly mixing them at room temperature with stirring, to subject the water and the hydrophilic solvent to natural drying.

The thus obtained glass-like gels, particularly gels formed using alkoxy silanes, are provided for practical use, whereby previous heat treatment at temperatures of about 100 to about 300°C for several hours is preferred from viewpoints of removing the remained unreacted substances and impurities, etc. and improving strength of holding a shape of the carrier per se. However, if the temperature for the heat treatment exceeds about 300°C (e.g., about 500°C), siloxane bonds increase within the glass-like gels to cause high degree of condensation so that the amount of hydroxy groups decreases, which is not preferred. At the heat treatment at about 300°C, silanol groups are hardly converted into siloxane bonds so that the decrease in the activity of the gel is hardly appreciated. .

The thus obtained glass-like gels are employed as carriers for immobilizing peptide-containing compounds thereto as they are or after they are shaped into a desired form (a plate-like, spherical or powdery form) in an appropriate manner. In case that the glass-like gels are formed in a layer on the inner surface of a glass tube or a stainless tube, a solution of the metal alkoxide in an aqueous solvent is coated onto the inner surface of the tube followed by hydrolysis as it is.

Coupling agents are usually reacted with the glass-like gels and peptide-containing compounds are immobilized to the resulting reaction product; thus, the bioreactors of the present invention are obtained.

Examples of the aforesaid peptide-containing compounds include biocatalysts such as enzymes, etc., antigens, antibodies, and the like.

More specific examples are enzymes such as glucose oxidase (GOD), cholesterol oxidase (CO), acyl CoA oxidase (ACO), 3-α-hydroxysteroid dehydrogenase (3α-HSD), glucose-6-phosphate dehydrogenase (G-6-PDH), glutamate-1-dehydrogenase (GIDH), creatininase, uricase, peroxidase, hexokinase (HK), urease, chlesterol esterase (CE), acyl CoA synthetase (ACS), etc.; antigens such as immunoglobulins, α-fetoprotein, haptoglobin, HBs antigen, etc.; antibodies such as $α_1$-fetoprotein, τ-globulin, etc.

As the coupling agents described above, silane coupling agents containing functional groups such as an amino group, a thiol group, an epoxy group, etc. which are conventional in the art can be employed. Specific examples include γ-amino-propyltriethoxysilane, γ-chloropropyl trimethoxysilane, vinyltriethoxysilane, γ-glycidoxypropyl trimethoxysilane, N-β-(aminoethyl)-γ-aminopropyl trimethoxysilane, etc. Cyanogen bromide can also be employed as the coupling agent. The coupling agents are generally bound to the hydroxy groups of the glass-like gels prior to immobilization. These coupling agents serve for immobilization of the peptide-containing compounds by introducing functional groups into the gels.

Reaction with the silane coupling agents are carried out under conditions known in the art. For example, in the case of using γ-aminopropyltriethoxysilane, the coupling agent is dissolved in water to make an about 10% aqueous solution. After adjusting pH to 3 to 5, the gels described above or shaped gels which were completely dried are added to the solution and mixed with heating to treat for several hours. Thereafter, the mixture is washed with water to remove the unreacted coupling agent to complete the reaction.

The binding with the peptide-containing compounds is generally performed utilizing the functional groups contained in the coupling agent described above. For example, in case that the glass-like gels having introduced therein many aminoalkyl groups through ester bonds at the hydroxy groups thereof using γ-aminopropyl triethoxysilane as the coupling agent are employed, Schiff's bases having an

aldehyde group is introduced into the aminoalkyl group described above using glutaraldehyde and an enzyme or the like is contacted therewith to form an additional Schiff's base between the aldehyde group and the amino group of the enzyme or the like, whereby immobilization is effected. In addition, immobilization can also be effected by diazotizing the aminoalkyl group, introducing an aromatic amino group thereto and immobilizing the peptide-containing compound thereto. Further, the immobilization can also be effected by directly forming a peptide bond between the aminoalkyl group and the peptide-containing compound using carbodiimide. Depending upon kind of the peptide-containing compounds, immobilization can be appropriately chosen. Enzymes or the like can also be immobilized directly by the coupling group or after appropriately modifying the coupling group, in a similar manner to when other coupling agents are employed.

The thus obtained bioreactors of the present invention are characterized in that the glass-like gels obtained without requiring any step of heat treatment at high temperatures are employed as carriers and any step of introducing hydroxy groups is required in immobilization. Accordingly, the present invention provides greatly reduced costs as compared to the prior art. Further, the bioreactors of the present invention have the activity higher than that of the prior art.

The high activity of the bioreactors is explained as follows. That is, the bioreactors of the present invention is characterized by the use of the glass-like gels comprising metal hydroxide compounds and/or condensation products thereof (particularly low degree condensation products) as carriers. In the case of using, e.g., $Si(OC_2H_5)_4$ as the metal alkoxide, a glass-like gel is obtained, the composition of which comprises $Si(OH)_4$ which is a hydrolysate of $Si(OC_2H_5)_4$ or its low degree condensation product. For this reason, this carrier contains many hydroxy groups as compared to a carrier using glass oxide obtained by treatment with an alkali so that the carrier markedly increase an amount of the peptide-containing compound to be immobilized; it is thus believed that the carrier would increase the activity as a bioreactor.

When a small quantity of hydrogen fluoride is contained in the aqueous solvent upon the aforesaid hydrolysis, it has also be found that a carrier having a further increased capability for immobilization can be obtained.

By such a treatment with hydrogen fluoride, gelation is accelerated and at the same time, a part of the hydroxy groups in the metal hydroxide compounds and/or condensation products thereof, which are the hydrolysis products of the metal alkoxides, is substituted with fluorine atoms to obtain glass-like gels having chemically bound fluorine atoms thereto. In this case, it is necessary to introduce a small quantity of fluorine atoms for the substitution and introduction. As this quantity, about 0.05 to about 1.0 mol is appropriate when expressed as a mole ratio to the raw metal alkoxide (1 mol) and about 0.2 mol is most preferred. With 0.05 mol or less, the effect due to the introduction of fluorine atoms is insufficient and such is not preferred. When the molar ratio exceeds 1.0 mol, the degree of substitution of the hydroxy groups in the metal hydroxide compounds and/or condensation products thereof is excessive and the number of the hydroxy groups capable of participating in the subsequent reaction is substantially decreased; such is thus not preferred. The gels obtained by the hydrolysis of the metal alkoxides, part of which is substituted with fluorine, provide further increased capability of immobilizing the peptide-containing compound, as compared to the gels to which no fluorine substitution is made. Ultra strong acids which generate hydrogen flouride can also be used in place of hydrogen fluoride. The glass-like gels having fluorine atoms are per se novel compound and useful as a carrier for various use.

As is shown in formula (I) below:

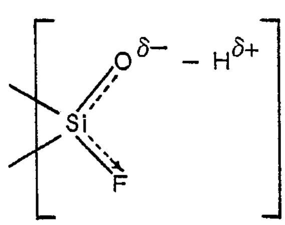

(I)

The effect due to the introduction of fluorine atom is believed to be because the degree of polarization of the hydroxy group in the silanol adjacent thereto would become large due to an electrophilic induction effect of the fluorine atom which has been introduced in a small quantity into the metal hydroxide compound or a condensation product thereof so that the hydrogen atom would become active and the reactivity would be more increased. Further, a microscopic observation indicates that the glass-like gels obtained in the presence of hydrogen fluoride upon the hydrolysis are highly porous. It is also believed that an effect of increasing surface areas due to such highly porous character would also contribute to the increased capability of immobilizing the peptide-containing compound.

In the case of making hydrogen fluoride co-present, it is preferred that a small quantity (0.05 to 1.0 mol) of hydrogen fluoride is added to an aqueous medium containing the metal alkoxide and, the formed alcohol, solvent, inorganic acid and unreacted hydrogen fluoride are allowed to evaporate off, while heating at about 80 to about 100°C and the formed gels are thoroughly dried at about 300°C.

It has also been found that, by accelerating the hydrolysis of the metal alkoxide in a dispersed state obtained by adding a solution of the metal alkoxide and hydrogen fluorid in an aqueous solvent to a liquid

hot medium incompatible but dispersible with the solution, spherical glass-like gels are obtained in good efficiency. In this case, hydrogen fluoride serves in the formation of spherical, particularly finely spherical glass-like gels by effectively promoting the hydrolysis per se, in addition to the effect described above. As a small quantity of hydrogen fluoride, it is appropriate to employ 0.1 to 1 mol per 1 mol of the metal alkoxide. Specific examples of liquid hot media which can be employed include hydrocarbon oils such as fluid paraffin, mineral oils, etc.; polyethylene glycol, polypropylene glycol, etc. In addition, various liquids such as synthetic oils, plant oils, etc. can also be employed. Dispersion in a liquid hot medium is generally effected by adding at once followed by stirring. In this case, it is preferred that a volume ratio for mixing the hydrogen fluoride-containing solution with the liquid hot medium be adjusted such that the former be not greater than 1/10 the later because spherical glass-like gels can be prepared in a yield of almost 100%. Further it is appropriate that the temperature of the liquid hot medium be set generally at 50 to 200°C. In the case of using, e.g., alkoxy silanes, the temperature of about 80°C is preferred. Dispersion in this medium is appropriately carried out generally by spraying of the solution described above.

By the dispersion in such a hot medium, the hydrolysis of the metal alkoxide vigorously proceeds so that dispersed grains are converted into gels in several ten minutes and glass-like gels of a spherical form are obtained in a dispersed state. Further, it may be conducted by spraying a solution of the metal alkoxide and hydrogen fluoride in an aqueous solvent, in a gaseous hot medium such as a hot air or nitrogen stream.

A particle size of the thus formed spherical glass-like gels greatly varies depending upon the viscosity of the metal alkoxide solution; in addition, the particle size also varies depending upon, e.g., the viscosity (temperature) of the liquid hot medium (there is a tendency that when the temperature of the liquid hot medium is low and the viscosity is relatively high, the particle size becomes large; when the temperature is high and the viscosity is relatively low, the particle size becomes small). Further, the particle size also varies more or less depending upon a time period required for dispersion and a dispersed state. However, by controlling various conditions, gels having a particle size of a large diameter of a µm order to a fine diameter of a µm order can be appropriately obtained.

By immobilizing the peptide-containing compound to the glass-like gels thus obtained by the fluorine-treatment using the coupling agent in a manner similar to the foregoing, the bioreactors of the present invention can be obtained. In the bioreactors, the amount of the peptide-containing compound immobilized is more increased and the activity is more increased, when compared to the bioreactors using as carriers the glass-like gels to which no fluorine treatment is made.

Hereafter the present invention will be described with reference to the examples.

## Example 1

(Preparation of Glass-like Gel: Step 1) .

A mixture of (pH 1) of 10 g of tetraethoxysilane $Si(OC_2H_5)_4$, 10 ml of ethanol, 3.8 ml of water and 1.4 ml of hydrochloric acid (1 N) was mixed at room temperature for about 1 hour with stirring until the system became homogeneous. The mixture was allowed to stand in the air for about 4 days to proceed with hydrolysis and the evaporation of water and ethanol. Then, the system was dried at about 120°C to obtain a glass-like gel of $Si(OH)_4$ type.

(Aminoalkylation: Step 2)

The gel obtained at Step 1 was ground in a glass mortar and passed through sieves to obtain beads having mean grain sizes of 60/80, 80/120 and 120/200.

A 5wt% γ-aminopropyltriethoxysilane aqueous solution was adjusted to pH 3.5 with 5 N hydrochloric acid, to 45 ml of which solution was added 5 g each of the aforesaid beads-like gel. Further the system was adjusted to pH 3.5. The mixture was charged in a four-necked glask equipped with a stirrer, a thermometer and a Dimroth's funnel. While maintaining the temperature at 75°C in a water bath, the reaction was carried out for 3 hours while vigorously stirring. After completion of the reaction, the beads were moved to a suction membrane filter to remove the unreacted γ-aminopropyltriethoxy silane with 1 l of distilled water. Then, the system was dried in a desicator to obtain three kinds of aminoalkylated glass-like gels having different grain sizes. The aminoalkylated gels are stored in a desicator.

The above-described aminoalkylated gels (beads-like) were immersed in each of a phosphate buffer solution (pH 7.0, 0.1M) of bifunctional glutaraldehyde (2.5 wt%). While reducing the pressure with an aspirator, the reaction was carried out for about 30 minutes with stirring. Subsequently, the reaction was carried out for about 30 minutes under normal pressure. The reaction temperature was 25°C. The reaction mixture was thoroughly washed with a phosphate buffer solution (0.1M, pH 7.0) followed by drying. By this treatment, a Schiff's base containing an aldehyde group is introduced at the terminal of the bound silane coupling group.

The gels were immersed in 1 mg/ml of a (glucose oxidase/phosphate buffer solution having pH 7.0). The reaction was carried out at 25°C for about 30 minutes firstly under reduced pressure while mildly stirring and then for 60 minutes under atmospheric pressure while mildly stirring to effect immobilization reaction. By this treatment, the amino group of glucose oxidase takes part in the reaction and forms an additional Schiff's base together with the aldehyde group of the carriers (gels). Thus, the glucose oxidase-immobilized gels (bioreactors) of the present invention were obtained, respectively.

(Activity Test)

The bioreactor of 200 mesh out of the beads-like bioreactors described above was subjected to an activity test with respect to the degree of immobilization, for the purpose of comparison with the prior art bioreactor. Firstly, 10 ml of a 1 wt% β-D-glucose solution (0.1 M, pH 7.0, phosphate buffer solution) was carefully poured into a beaker into which 1 g of the aforesaid immobilized glass beads of the present invention. The mixture was mildly stirred for about 15 minutes to cause a reaction. From the reaction mixture, 9 ml was taken and 1 ml of a 5% potassium iodide solution was added thereto. By this treatment, $I^-$ was oxidized to $I_2$ (aq) by hydrogen peroxide released in the enzyme reaction. This $I_2$ was reduced by the voltametry method. By measuring the reduction wave, the $I_2$ content can be determined and thus, the degree of the enzyme immobilized in the bioreactor, i.e., the degree of activity, is determined.

The results obtained by the voltametry method of a conventional bioreactor obtained in a manner similar to the foregoing using the prior art glass as a carrier and the bioreactor of the present invention are shown in Table 1.

TABLE 1

| | Carrier used (beads) | Reduction Current* (mA) |
|---|---|---|
| Example | Glass*like Gel | 0.7 |
| Comparative Example | Commercially Available Glass | 0.5 |

* 0.53 VSNHE

The reduction current is obtained by subtracting a blank value.

The potential scanning speed is 20 mV/sec.

From the results above, it is understood that the bioreactor of the present invention provides obviously higher enzyme activity than the conventional bioreactor using the prior art glass as a carrier.

Example 2

The bioreactor of the present invention was prepared in a manner similar to Example 1 except that enzymes used were different.

The enzymes used and a unit of each of the enzymes used for immobilization per 1 mg of a carrier are shown in the table below.

| Kind of Enzyme | Unit |
|---|---|
| Uricase | 0.25 u |
| Cholesterol Oxidase | 0.01 U |
| Acyl CoA Oxidase | 0.1 U |
| Urease | 1 U |
| Cholesterol Esterase | 0.03 U |
| Creatininase | 1 U |
| Acyl CoA Synthetase | 0.05 U |

Example 3

(Preparation of Fluorine-Substituted Glass-like Gel)

A mixture (pH about 1) of 0.52 mol of tetraethoxysilane $Si(OC_2H_5)_4$, 1.72 mol of ethanol, 1.82 mol of water, 0.028 mol of hydrochloric acid was mixed for several ten minutes at room temperature with stirring until the system became homogeneous, followed by 0.058 to 0.115 mol of hydrogen fluoride was added with stirring.

Then the mixture was heated in a water bath of 80°C for 3 days. By evaporating the ethanol formed during the hydrolysis and the remained water hydrochloric acid and a trace amount of the unreacted hydrogen fluoride, about 30 g of a glass-like gel was obtained.

(Aminoalkylation)

The thus obtained glass-like gel was ground to obtain beads of 120/200 mesh.

A 5wt% γ-aminopropyltriethoxysilane aqueous solution was adjusted pH to 3.5 with 5 N hydrochloric acid, to 100 ml of which solution was added 5 g each of the aforesaid beads-like gel. Further the system was adjusted to pH 3.5. The mixture was charged in a four-necked glask equipped with a stirrer, a thermometer and a Dimroth's funnel. While maintaining the temperature at 75°C in a water bath, the reaction was carried out for 3 hours while vigorously stirring. After completion of the reaction, the beads were moved to a suction membrane filter to remove the unreacted γ-aminopropyltriethoxy silane with 1 l of distilled water. Then, the system was dried in a desicator to obtain three kinds of aminoalkylated glass-like gels having different grain sizes. The aminoalkylated gels are stored in a desicator.

(Immobilization of Enzyme)

The above-described aminoalkylated gels (beads-like) were immersed in each of a phosphate buffer solution (0.1 M, pH 7.0) of bifunctional glutaraldehyde (2.5 wt%). While reducing the pressure with an aspirator, the reaction was carried out for about 30 minutes with stirring. Subsequently, the reaction was carried out for about 30 minutes under normal pressure. The reaction temperature was 30°C. The reaction mixture was thoroughly washed with a phosphate buffer solution having pH 7.0 followed by drying. By this treatment, a Schiff's base containing an aldehyde group is introduced.

The gels were immersed in 10 ml of a 1 mg/ml of a glucose oxidase/phosphate buffer solution having pH 7.0 (0.1 M). The reaction was carried out at 25°C for about 30 minutes firstly under reduced pressure while mildly stirring and then for 60 minutes under normal pressure while mildly stirring to effect immobilization reaction. By this treatment, the amino group of glucose oxidase takes part in the reaction and forms an additional Schiff's base together with the aldehyde group of the carriers (gels). Thus, the glucose oxidase-immobilized glass-like gels (immobilized enzymes) of the present invention were obtained, respectively.

The thus obtained immobilized enzyme was examined with respect to the activity with the passage of time by means of polarography. The results are shown in Fig. 1, wherein the measurement conditions are as follows:

Test solution: β-D(+)-glucose, 300 mg/dl of 0.1 M, ph 7.0 phosphate buffer

Measurement Temperature: 28°C

Storage Temperature: 4°C

The measurement was performed by means of polarography using a dropping mercury electrode, after mixing 1 g of the immobilized enzyme with 11 ml of the test solution for 5 minutes with stirring and separating 10 ml of the mixture by filtration using a membrane filter. The half wave potential of $H_2O_2$ generated by the enzyme reaction was made −0.85 V (vs) Ag/AgCl electrode.

No change was observed in the activity even 2 months and the half after.

On the other hand, an immobilized enzyme was obtained in a similar manner using a glass-like gel prepared in a similar manner except for using no hydrogen fluoride. Comparison was made with the thus obtained immobilized enzyme. The results are shown in Fig. 2, wherein (1) indicates the example and (2) indicates a comparative example. As is shown in Fig. 2, it is understood that the above-described immobilized enzyme possesses the activity of about 2.5 times that of the similarly immobilized enzyme to which no fluorine atom has been introduced.

Example 4

Glass-like gels were obtained in a manner similar to Example 3, respectively, except that the reaction was carried out under the condition that the mole ratios of $HF/Si(OC_2H_5)_4$ were in the range of 0.1 to 0.5. To each of these carriers, glucose oxidase was immobilized in a manner similar to the foregoing to obtain immobilized enzymes. The relationship between the activities of these immobilized enzymes and the mole ratios described above is shown in Fig. 3. The measurement of the activities was also performed as described above.

As shown in the figure, it is understood that the enzyme activity is affected by the content of hydrogen fluoride in preparing the carriers and in particular, the maximum activity (about 7 times that of a blank test) appears at about 0.2 in the mole ratio. It is also understood that the addition of inorganic acids such as hydrochloric acid, etc. in the preparation of the carriers (plot O) is more suitable than no addition (plot Δ).

Further SEM (Scanning Electron Microscope) (× 20000) of the glass-like gels obtained in the mole ratios of 0.1 and 0.5 indicate that the porosity at the surfaces is higher than that of the system to which no fluorine has been introduced.

Further, an analytical chart of each of the porous glass-like gel carriers (glass beads-like) obtained in Example 4 by ESCA (Electron Spectrometry for Chemical Analysis or x ray photoelectron analysis) is shown in Fig. 4, wherein A represents a scanning speed of 2 eV/sec and B represents a scanning speed of 1 eV/sec. The conditions for the measurement of ESCA are as follows:

Target: Mg

Accelerated Potential: 8 kV

Filament: 30 mA

Ar Etching Conditions: accelerated potential 2 kV emission 30 mA time 15 minutes

From the fact that the peak due to fluorine atoms is observed at the binding energy of about 700 eV, it is understood that the fluorine atoms are chemically bound. Further, Table 2 is a comparison between a bulk state and a powder state with respect to the specific intensity of the peaks of $F_{1s}$ and $Si_{2s}$ in the ESCA spectrum of the carriers when $HF/Si(OC_2H_5)_4$ was 0.1 in a mole ratio. From the fact that the specific intensities of both are approximate to each other, it is understood that not only the surface but also the whole of the carriers is converted into a fluorine compound.

TABLE 2

| Carrier Sample | Specific Intensity of $F_{1s}/Si_{2s}$ |
|---|---|
| Bulk State | 0.36 |
| Powder State | 0.47 |

Example 5

1 mole of tetraethoxysilane 3,557 mols of ethanol, 3.759 mols of water and 0.28 mol of hydrochloric acid were in turn mixed and the mixture was vigorously stirred to obtain a homogeneous sol. The resulting sol was mixed with 0.2 mol of hydrogen fluoride and a solid glass-like gel was obtained in a manner similar to Example 3.

With the gel, influences due to heat treatments at 300°C and 500°C were examined. That is, 500 mg each of the carrier ground to a 120/200 mesh was heated at 300°C and 500°C, respectively, in an electric furnace. Thereafter, aminoalkylation and immobilization of glucose oxidase were performed as in Example 3. The rate of immobilization (which was obtained by determining the protein contents in an enzyme solution prior to the immobilization and in an enzyme solution after the immobilization by the Lowry method and substracting the difference in both) of each of the thus obtained immobilized enzymes was measured.

The results are shown in Table 3, together with comparative examples.

TABLE 3

| Carrier | Carrier treated with no hydrogen fluoride | Carrier without heat treatment | Carrier heated at 300°C | Carrier heated at 500°C |
|---|---|---|---|---|
| Ratio of Immobilization (%) | 18 | 95 | 90 | 45 |

On the other hand, thermal weight analysis (TG) and differential thermal weight analysis (DTG) were performed with each of carriers prior to the aminoalkylation and the immobilization (carrier treated with no hydrogen fluoride . . . (A), carrier with no heat treatment . . . (B), carrier heat treated at 300°C . . . (C)). The results are shown in Fig. 5 and Fig. 6, respectively.

As shown in the figures, it is understood that the silanol groups of the carrier treated with hydrogen fluoride are little changed to siloxane bonds with the heat treatment at about 300°C and a moisture content absorbed by the carrier treated with hydrogen fluoride is extremely small.

Example 6

To 1 mol of tetraethoxysilane $Si(OC_2H_5)_4$, 3.8 mols of water, 3.6 mols of ethylalcohol and 0.28 mol of hydrochloric acid were in turn added and the mixture was vigorously stirred to obtain a solution of tetraethoxysilane in an aqueous solvent. While stirring, 0.1 to 1.0 mol of hydrogen fluoride was added to the solution. The mixture was added at once to 5000 ml of fluid paraffin previously heated to about 80°C. The mixture was stirred to disperse the above described solution therein. The dispersed particles were gelled in about 30 minutes to obtain micro-spherical glass-like gel in a dispersed state.

The spherical glass-like gel described above was filtered using a membrane filter to separate from the medium. After the glass-like gel was repeatedly washed with acetone, the gel was dried at 100°C. The spherical glass-like gel was a uniform spherical form as shown in Fig. 7, almost more than 95% of which were relatively uniform in a pore diameter of about 250 to about 500 µm.

The glass-like gel was treated in a manner similar to Example 3 to obtain a micro-spherical, immobilized enzyme.

Example 7

The bioreactors were prepared in a manner similar to Example 3 except that different enzymes were employed. The enzymes employed and a unit of each of the enzymes used for immobilization per 1 mg of a carrier are shown below.

| Kind of Enzyme | Unit (per 1 mg) |
|---|---|
| Cholesterol Oxidase | 0.01 U |
| Acyl CoA oxidase | 0.1 U |
| 3α-Hydroxysteroid Dehydrogenase | 0.05 U |
| Glucose-6-phosphate Dehydrogenase | 1 U |
| Glutamate-1-Dehydrogenase | 10 U |
| Peroxidase | 15 U |
| Hexokinase | 2 U |
| Urease | 1 U |
| Cholesterol Esterase | 0.03 U |
| Acyl CoA Synthetase | 0.05 U |
| (Co-Immobilization) | |
| Hexokinase/Glucose-6-phosphate Dehydrogenase | 2 U/1 U |

Example 8.

The bead-like, urease-immobilized enzyme and glutamate-1-dehydrogenase-immobilized enzyme obtained in Example 7 were filled up in a stainless tube having an inner diameter of 2 mm and a length of 5 cm, respectively. These immobilized enzyme columns were connected in series to perform assay for urea.

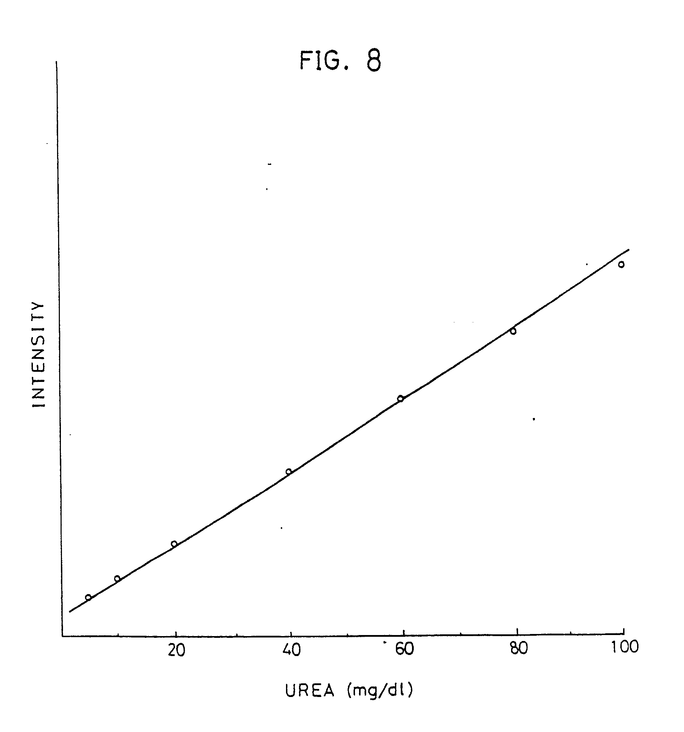

The conditions for the assay were as shown below and the relative activities of the enzymes were measured varying the urea content. The results are shown in Fig. 8.

Mobile phase: solution of 0.1 mM NADPH and 0.83 mM 2-oxoglutarate

Flow rate: 1.09 ml/m

Detector: fluorophotometer (excitation wavelength, 360 nm detection wavelength, 450 nm)

As shown in Fig. 8, good linear relationship was obtained between the urea content and the relative activity of the enzyme.

**Claims**

1. A bioreactor comprising a peptide-containing compound chemically bound to a carrier, characterised in that the carrier is a glass-like gel comprising a metal hydroxy compound and/or a condensation product thereof obtainable by hydrolysis of a metal alkoxide and/or of an alkoxysilane.

2. A bioreactor comprising a peptide-containing compound chemically bound to a carrier, characterised in that the carrier is a glass-like gel comprising a metal hydroxy compound and/or a condensation product thereof, the hydroxy groups of which are partially substituted by fluorine atoms, said carrier being obtainable by hydrolysis of a metal alkoxide and/or of an alkoxysilane and substitution of some of the resulting hydroxy groups by fluorine atoms.

3. A bioreactor according to claim 1 or 2, characterised in that the metal alkoxide is a lower alkoxytitanium, a lower alkoxy aluminium or a lower alkoxyvanadium, optionally in admixture with a lower alkoxysodium, or a mixture thereof.

4. A bioreactor according to any of the preceding claims, characterised in that the lower alkoxysilane is tetraethoxysilane.

5. A bioreactor according to any of the preceding claims, characterised in that the immobilization of the peptide-containing compound on the carrier is carried out after treatment of the carrier with a coupling agent.

6. A bioreactor according to claim 5, characterised in that the coupling agent is γ-aminopropyltriethoxysilane, γ-chloropropyltrimethoxysilane, vinyltriethoxysilane, α-glycidoxypropyltrimethoxysilane or N-β-(aminoethyl)-α-aminopropyltrimethoxysilane.

7. A bioreactor according to any of the preceding claims, characterised in that the peptide containing compound is an enzyme, an antigen or an antibody.

8. A bioreactor according to claim 7, characterised in that the enzyme is glucose oxidase, cholesterol oxidase, acyl CoA oxidase, 3α-hydroxysteroid dehydrogenase, glucose-6-phosphate dehydrogenase, glutamate-1-dehydrogenase, peroxidase, hexokinase, urease, cholesterol esterase or acyl CoA synthetase.

9. A bioreactor according to any of the preceding claims, characterised in that it is formed with a spherical shape.

10. A bioreactor according to any of claims 1 to 8, characterised in that it is formed with a fine particle shape.

11. A bioreactor according to any of claims 1 to 8, characterised in that it is formed as a film.

12. A bioreactor according to claim 11, characterised in that the film is formed on the inner surface of a glass tube or a stainless steel tube.

13. A bioreactor according to any of claims 2 to 12, characterised in that the substitution of hydroxyl groups by fluorine atoms is achieved by the use of 0.05—1.0 mol of a fluorine compound per 1 mole of metal alkoxide.

14. A process for preparing a bioreactor, characterised in that a metal alkoxide and/or an alkoxysilane is hydrolysed in an aqueous medium to form a glass-like gel of a metal hydroxy compound and/or a condensation product thereof, treating it with a coupling agent, optionally after appropriately forming into a suitable shape, and then immobilizing a peptide-containing compound on the resulting material.

15. A process according to claim 14, characterised in that fluorine atoms are incorporated into the glass-like gel.

16. A process according to claim 15, characterised in that fluorine atoms are incorporated by the use of hydrofluoric acid in a amount of 0.05—1.0 mol per 1 mol of the metal alkoxide.

17. A process according to any of claims 14 to 16, characterised in that the aqueous medium is a volatile hydrophilic organic solvent containing water.

18. A process according to claim 17, characterised in that the aqueous medium is an aqueous lower alcohol.

19. A process according to any of claims 14 to 18, characterised in that the hydrolysis is carried out at a pH of 1—3 and at a temperature of from ambient temperature to 100°C.

20. A process according to any of claims 15 to 19, characterised in that fluorine atoms are incorporated by adding hydrofluoric acid to an aqueous solution of a metal alkoxide, and dispersing the resulting solution in a hot liquid medium which is incompatible with but dispersible in the aqueous solution while the hydrolysis of the metal alkoxide is proceeding.

21. A process according to claim 20, characterised in that the temperature of the hot liquid medium is 50 to 200°C.

**Patentansprüche**

1. Bioreaktor, umfassend eine Peptid enthaltende Verbindung, chemisch gebunden an einen Träger, dadurch gekennzeichnet, daß der Träger ein glasartiges Gel ist, umfassend eine Metallhydroxyverbindung und/oder ein Kondensationsprodukt davon, die bzw. das durch Hydrolyse eines Metallalkoxids und/oder eines Alkoxysilans erhalten werden kann.

2. Bioreaktor, umfassend eine Peptid enthaltende Verbindung, chemisch gebunden an einen Träger, dadurch gekennzeichnet, daß der Träger ein glasartiges Gel ist, umfassend eine Metallhydroxyverbindung und/oder ein Kondensationsprodukt davon, deren bzw. dessen Hydroxygruppen teilweise durch Fluoratome substituiert sind, und der Träger durch Hydrolyse eines Metallalkoxids und/oder eines Alkoxysilans und durch Substitution einiger der erhaltenen Hydroxygruppen durch Fluoratome erhalten werden kann.

3. Bioreaktor nach Anspruch 1 oder 2, dadurch gekennzeichnet, daß das Metallalkoxid ein Niederalkoxytitan, ein Niederalkoxyaluminium oder ein Niederalkoxyvanadium, gegebenenfalls im Gemisch mit einem Niederalkoxynatrium, oder einem Gemisch davon ist.

4. Bioreaktor nach einem der vorhergehenden Ansprüche, dadurch gekennzeichnet, daß das Niederalkoxysilan Tetraethoxysilan ist.

5. Bioreaktor nach einem der vorhergehenden Ansprüche, dadurch gekennzeichnet, daß die Immobilisierung der Peptid enthaltenden Verbindung auf dem Träger nach der Behandlung des Trägers mit einem Kupplungsmittel vorgenommen wird.

6. Bioreaktor nach Anspruch 5, dadurch gekennzeichnet, daß das Kupplungsmittel γ-Aminopropyltriethoxysilan, γ-Chlorpropyltrimethoxysilan, Vinyltriethoxysilan, α-Glycidoxypropyltrimethoxysilan oder N-β-(Aminoethyl)-α-aminopropyltrimethoxysilan ist.

7. Bioreaktor nach einem der vorhergehenden Ansprüche, dadurch gekennzeichnet, daß die Peptid enthaltende Verbindung ein Enzym, ein Antigen oder ein Antikörper ist.

8. Bioreaktor nach Anspruch 7, dadurch gekennzeichnet, daß das Enzym Glucose-Oxidase, Cholesterin-Oxidase, Acyl-CoA-Oxidase, 3α-Hydroxysteroid-Dehydrogenase, Glucose-6-phosphat-Dehydrogenase, Glutamat-1-Dehydrogenase, Peroxidase, Hexokinase, Urease, Cholesterin-Esterase oder Acyl-CoA-Synthetase ist.

9. Bioreaktor nach einem der vorhergehenden Ansprüche, dadurch gekennzeichnet, daß er kugelig geformt ist.

10. Bioreaktor nach einem der Ansprüche 1 bis 8, dadurch gekennzeichnet, daß er die Form feiner Teilchen hat.

11. Bioreaktor nach einem der Ansprüche 1 bis 8, dadurch gekennzeichnet, daß er die Form eines Filmes hat.

12. Bioreaktor nach Anspruch 11, dadurch gekennzeichnet, daß der Film auf der Innenfläche eines Glasrohrs oder eines Rohres aus rostfreiem Stahl aufgebracht ist.

13. Bioreaktor nach einem der Ansprüche 2 bis 12, dadurch gekennzeichnet, daß die Substitution der Hydroxylgruppen durch Fluoratome mit Hilfe von 0,05 bis 1,0 mol einer Fluorverbindung je 1 mol Metallalkoxid durchgeführt wird.

14. Verfahren zur Herstellung eines Bioreaktors, dadurch gekennzeichnet, daß ein Metallalkoxid und/oder ein Alkoxysilan in einem wäßrigen Medium hydrolysiert wird unter Bildung eines glasartigen Gels einer Metallhydroxyverbindung und/oder eines Kondensationsprodukts davon, dieses mit einem Kupplungsmittel behandelt wird, gegebenenfalls nach geeignetem In-Form-Bringen, und dann eine Peptid enthaltende Verbindung auf dem erhaltenen Material immobilisiert wird.

15. Verfahren nach Anspruch 14, dadurch gekennzeichnet, daß Fluoratome in das glasartige Gel eingebaut werden.

16. Verfahren nach Anspruch 15, dadurch gekennzeichnet, daß Fluoratome eingebaut werden durch Einsatz von Fluorwasserstoffsäure in einer Menge von 0,05 bis 1,0 mol je 1 mol Metallalkoxid.

17. Verfahren nach einem der Ansprüche 14 bis 16, dadurch gekennzeichnet, daß das wäßrige Medium ein Wasser enthaltendes flüchtiges hydrophiles organisches Lösungsmittel ist.

18. Verfahren nach Anspruch 17, dadurch gekennzeichnet, daß das wäßrige Medium ein wäßriger niederer Alkohol ist.

19. Verfahren nach einem der Ansprüche 14 bis 19, dadurch gekennzeichnet, daß die Hydrolyse bei einem pH-Wert von 1 bis 3 und einer Temperatur von Umgebungstemperatur bis 100°C durchgeführt wird.

20. Verfahren nach einem der Ansprüche 15 bis 19, dadurch gekennzeichnet, daß die Fluoratome eingebaut werden durch Zugabe von Fluorwasserstoffsäure zu einer wäßrigen Lösung eines Metallalkoxids und Dispergieren der erhaltenen Lösung in einem heißen flüssigen Medium, das mit der wäßrigen Lösung unverträglich, aber darin dispergierbar ist, während die Hydrolyse des Metallalkoxids abläuft.

21. Verfahren nach Anspruch 20, dadurch gekennzeichnet, daß die Temperatur des heißen flüssigen Mediums 50 bis 200°C beträgt.

## Revendications

1. Bioréacteur comprenant un composé contenant un ou des peptides liés chimiquement à un support, caractérisé en ce que le support est un gel ressemblant à du verre comprenant un composé hydroxy méthallique et/ou un produit de condensation de celui-ci, pouvant être obtenu par hydrolyse d'un alcoolate métallique et/ou d'un alcoxysilane.

2. Bioréacteur comprenant un composé contenant un peptide lié chimiquement à un support, caractérisé en ce que ce support est un gel ressemblant à du verre comprenant un composé hydroxy métallique et/ou un produit de condensation de celui-ci, dont les groupes hydroxy sont partiellement substitutés par des atomes de fluor, ce support pouvant être obtenu par hydrolyse d'un alcoolate métallique et/ou d'un alcoxysilane et substitution de certains des groupes hydroxy résultants par des atomes de fluor.

3. Bioréacteur suivant la revendication 1 ou 2, caractérisé en ce que l'alcoolate métallique est un alcoxy(inférieur)titane, un alcoxy(inférieur)aluminium ou un alcoxy(inférieur)vanadium, éventuellement en mélange avec un alcoxy(inférieur)sodium ou un mélange de ceux-ci.

4. Bioréacteur suivant l'une quelconque des revendications précédentes, caractérisé en ce que l'alcoxysilane inférieur est le tétrahydroxysilane.

5. Bioréacteur suivant l'une quelconque des revendications précédentes, caractérisé en ce que l'immobilisation du composé contenant un peptide sur le support est effectuée après traitement du support avec un agent de couplage.

6. Bioréacteur suivant la revendication 5, caractérisé en ce que l'agent de couplage est le γ-aminopropyltriéthoxysilane, le γ-chloropropyltriméthoxysilane, le vinyltriéthoxysilane, l'α-glycidoxy-propyltriméthoxysilane ou le N-β-(aminoéthyl)-α-aminopropyltriméthoxysilane.

7. Bioréacteur suivant l'une quelconque des revendications précédentes, caractérisé en ce que le composé contenant un peptide est une enzyme, un antigène ou un anticorps.

8. Bioréacteur suivant la revendication 7, caractérisé en ce que l'enzyme est choisie parmi les glucose oxydase, cholestérol oxydase, acyl CoA oxydase, 3α-hydroxystéroïde deshydrogénase, glucose-6-phosphate, deshydrogénase, glutamate-1-deshydrogénase, peroxydase, hexokinase, uréase, cholestérol estérase ou acyl CoA synthétase.

9. Bioréacteur suivant l'une quelconque des revendications précédentes, caractérisé en ce qu'il est mis sous une forme sphérique.

10. Bioréacteur suivant l'une quelconque des revendications 1 à 8, caractérisé en ce qu'il est mis sous forme de fine particule.

11. Bioréacteur suivant l'une quelconque des revendications 1 à 8, caractérisé en ce qu'il est mis sous forme d'un film.

12. Bioréacteur suivant la revendication 11, caractérisé en ce que le film est formé sur la surface intérieure d'un tube en verre ou d'un tube en acier inoxydable.

13. Bioréacteur suivant l'une quelconque des revendications 2 à 12, caractérisé en ce que la substitution des groupes hydroxy par des atomes de fluor est réalisée à l'aide de 0,05 à 1,0 mole d'un composé du fluor par mole d'alcoolate métallique.

14. Procédé pour préparer un bioréacteur, caractérisé en ce qu'un alcoolate métallique et/ou un alcoxysilane sont hydrolysés dans un milieu aqueux pour former un gel ressemblant à du verre d'un composé hydroxy métallique et/ou d'un produit de condensation de celui-ci, ce gel est traité avec un agent de couplage, éventuellement après avoir été convenablement sous une forme appropriée et ensuite un composé contenant un peptide est immobilisé sur le matériau résultant.

15. Procédé suivant la revendication 14, caractérisé en ce que des atomes de fluor sont incorporés dans le gel ressemblant à du verre.

16. Procédé suivant la revendication 15, caractérisé en ce que les atomes de fluor sont incorporés à l'aide d'acide fluorhydrique en une quantité de 0,05 à 1,0 mole par mole d'alcoolate métallique.

17. Procédé suivant l'une quelconque des revendications 14 à 16, caractérisé en ce que le milieu aqueux est un solvant organique hydrophile volatil contenant de l'eau.

18. Procédé suivant la revendication 17, caractérisé en ce que le milieu aqueux est un alcool inférieur aqueux.

19. Procédé suivant l'une quelconque des revendications 14 à 18, caractérisé en ce que l'hydrolyse est effectuée à un pH de 1 à 3 et à une température allant de la température ambiante à 100°C.

20. Procédé suivant l'une quelconque des revendications 15 à 19, caractérisé en ce que des atomes de fluor sont incorporés par addition d'acide fluorhydrique à une solution aqueuse d'un alcoolate métallique et dispersion de la solution résultante dans un milieu liquide chaud qui est incompatible avec la solution aqueuse, mais dans lequel elle peut se disperser, alors que se déroule l'hydrolyse de l'alcoolate métallique.

21. Procédé suivant la revendication 20, caractérisé en ce que la température du milieu liquide chaud est de 50 à 200°C.

FIG. 1

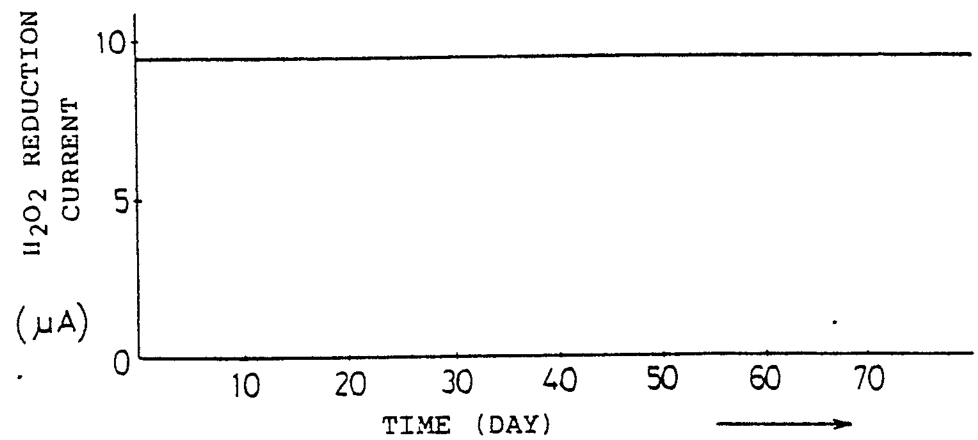

FIG. 2

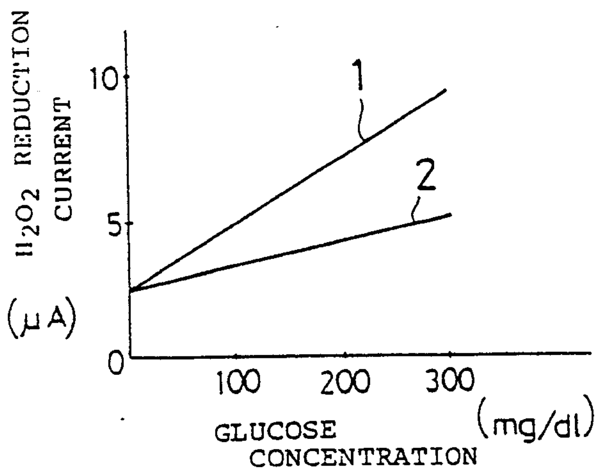

$H_2O_2$ REDUCTION CURRENT
(μA)
10
5
0
1
2
100
200
300
GLUCOSE CONCENTRATION
(mg/dl)

FIG. 3

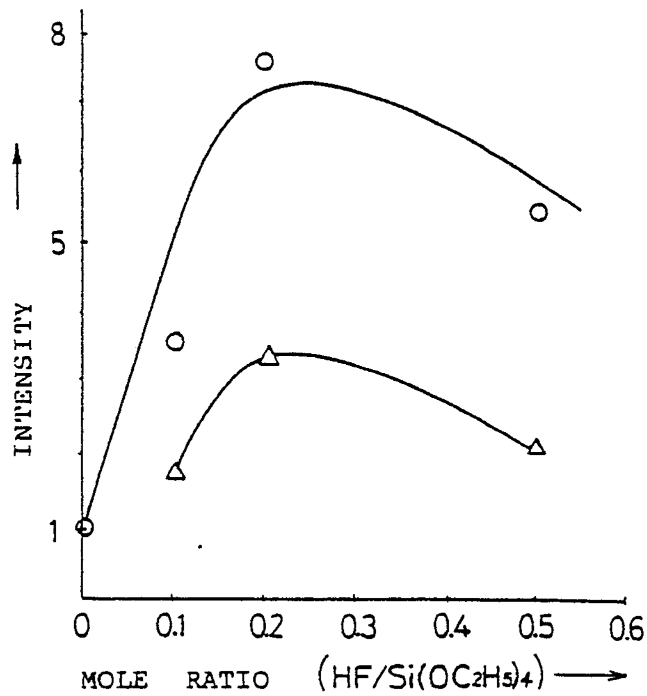

FIG. 4

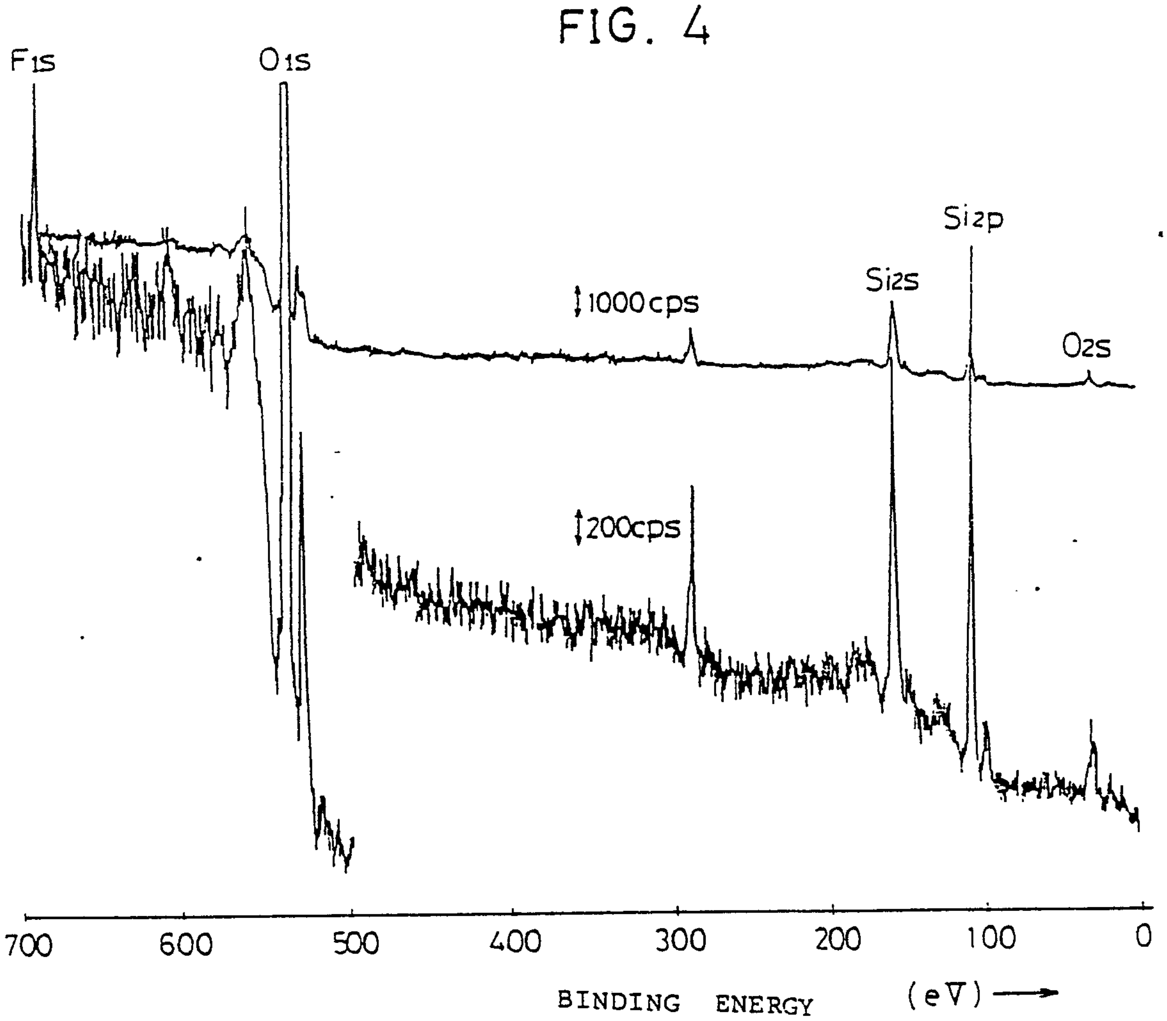
F1s
O1s
Si2p
Si2s
1000cps
O2s
200cps
700
600
500
400
300
200
100
0
BINDING ENERGY (eV)

FIG. 5

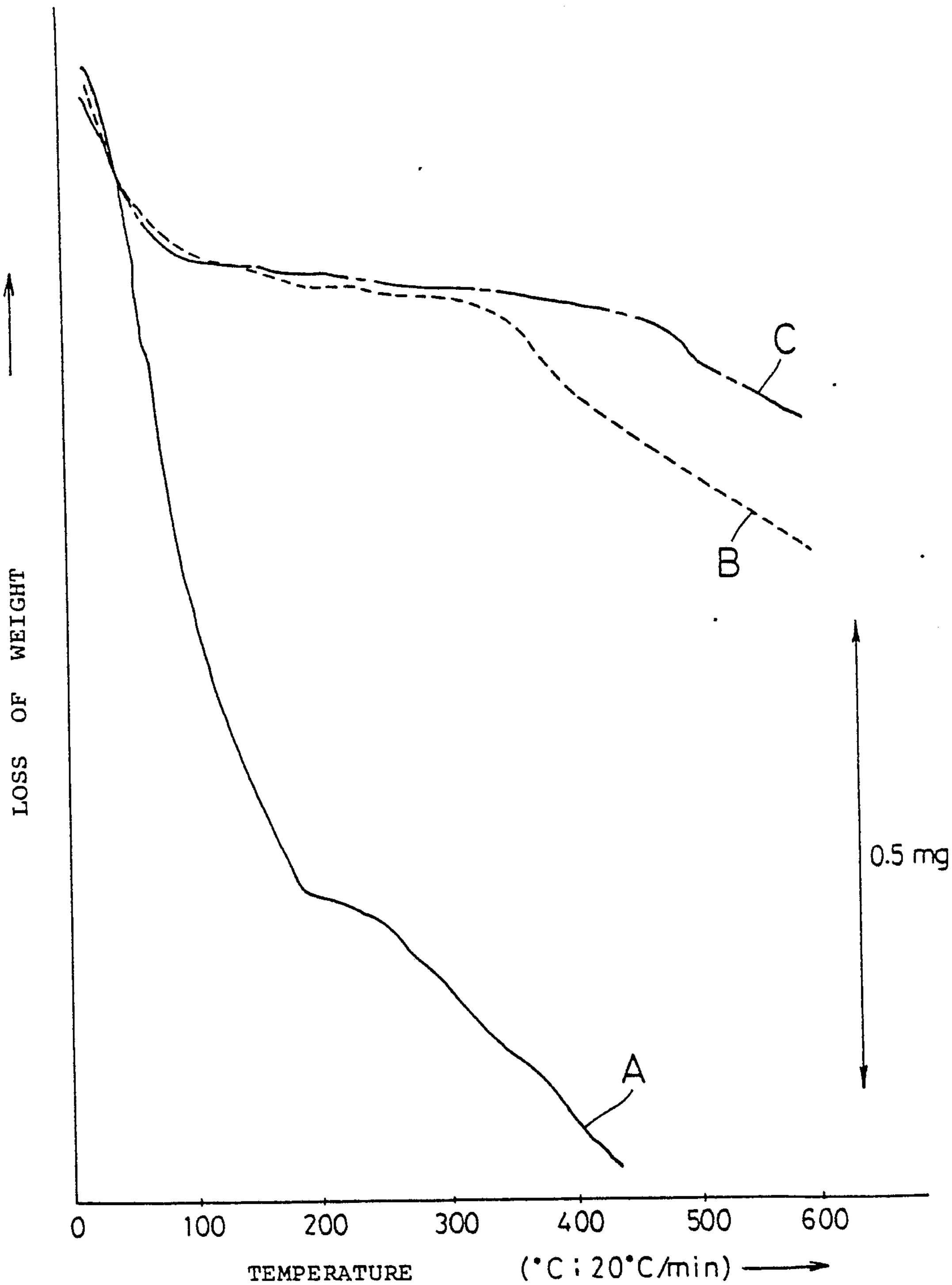
LOSS OF WEIGHT
C
B
A
0.5 mg
0
100
200
300
400
500
600
TEMPERATURE
(°C ; 20°C/min)

FIG. 6

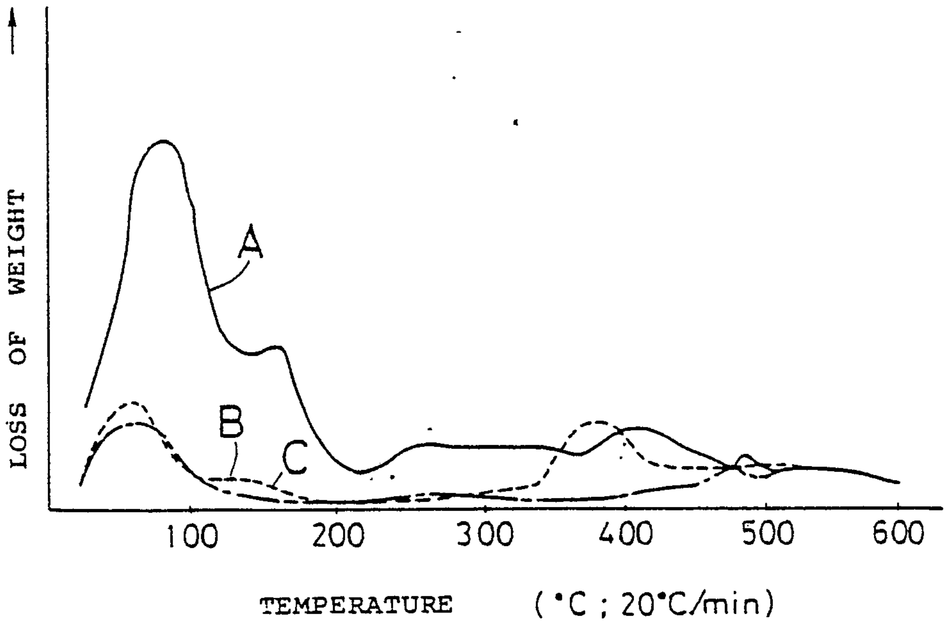
LOSS OF WEIGHT
A
B
C
100
200
300
400
500
600
TEMPERATURE (°C ; 20°C/min)

FIG 7

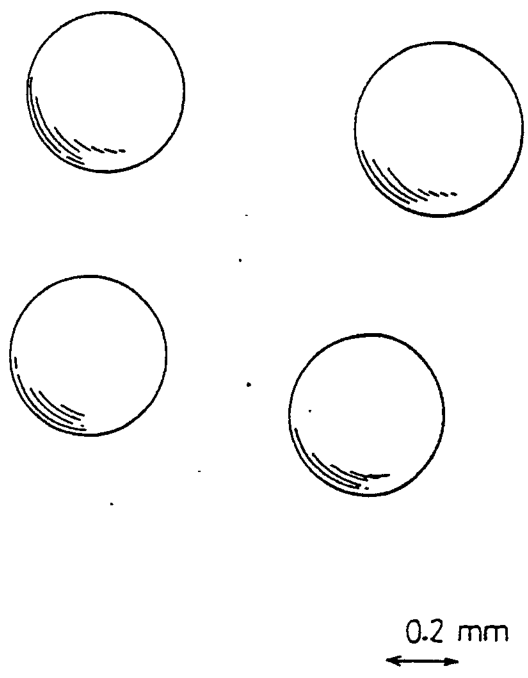

FIG. 8